Europäisches Patentamt

European Patent Office

Office européen des brevets

(11) Publication number: **0 261 676**
**A2**

# (12) EUROPEAN PATENT APPLICATION

(21) Application number: 87113958.0

(22) Date of filing: 24.09.87

(51) Int. Cl.⁴: **C12M 1/22 , C12M 1/12**

(30) Priority: 26.09.86 US 912436

(43) Date of publication of application:
**30.03.88 Bulletin 88/13**

(84) Designated Contracting States:
**AT BE CH DE ES FR GB GR IT LI NL SE**

(71) Applicant: **Becton, Dickinson and Company**
**One Becton Drive**
**Franklin Lakes New Jersey 07417-1880(US)**

(72) Inventor: **Monthony, James F.**
**13 Thaxton Court Baltimore County**
**Timonium Maryland 21093(US)**
Inventor: **Sapitowicz, Robert**
**604 Camelot Drive**
**Hartford County Bel Air Maryland 21014(US)**
Inventor: **Corasaniti, Virginia**
**2707 Kildaire Drive Baltimore City County**
**Baltimore Maryland 21234(US)**
Inventor: **Clisham, Patricia**
**2814 Inglewood Avenue Baltimore City**
**County**
**Baltimore Maryland 21234(US)**

(74) Representative: **von Kreisler, Alek,**
**Dipl.-Chem. et al**
**Deichmannhaus am Hauptbahnhof**
**D-5000 Köln 1(DE)**

(54) A method and device for concentration of particles on a solid surface.

(57) A method and device for concentration of particles present in a fluid sample located on the surface of a solid material. The concentration device is a body having a peripheral flange surrounding a depending chamber. The chamber has side walls and a bottom wall having an opening therein. An absorbent material is disposed within the chamber and a membrane is disposed over the opening in the bottom wall. The membrane is pervious to fluids but is impervious to the particles. Means are provided for locating the membrane in proximity to the surface of the solid material.

EP 0 261 676 A2

# A METHOD AND DEVICE FOR CONCENTRATION OF PARTICLES ON A SOLID SURFACE

## Background of the Invention

### Field of the Invention:

The present invention is directed to a method and device for the concentration of particles present in a fluid sample located on the surface of a solid material. More particularly, the present invention is directed to the concentration of microorganisms in a fluid sample wherein the fluid sample is present on a solid nutrient medium dispersed in a petri dish.

### Prior Art:

Numerous attempts have been made to increase the concentration of microorganisms present in a sample prior to inoculation of plated media for growth. [1-9]Centrifugation of urine[1] is used to concentrate bacteria. Bacterial contamination of water[2-3] and food[9] samples is assessed after collection of bacteria from dilute samples on a membrane filter. Membrane filtration, alone [8-9] and in combination with centrifugation [5,6] has been applied to the problem of detection of bacteria in blood culture samples in the clinical microbiology laboratory. A specialized centrifugation based product[10] is marketed for this application. Recently, Sage, et al[7] described a device and method to enhance results obtained with the membrane filtration approach. This method did not allow culture of the concentrated organisms.

Blood culture samples represent an excellent example of the need to detect and culture microorganisms present at low levels. The presence of bacteria in blood is a serious medical condition requiring rapid treatment. The isolation, the identification and the determination of antibiotic susceptibility of the infectious agent is critical. One object of the present invention is to increase greatly the ability of the clinical laboratory to recover and grow organisms present in low concentration from samples such as blood cultures. A further object of this invention is to remove the need to utilize centrifugation as a way to concentrate microorganisms from clinical samples. Numerous time consuming safety measures are involved in the use of centrifugation of infectious materials (or potentially infectious samples) in a clinical laboratory.[11]

## REFERENCES

1. Bradley, G.M. and Benson, E.S. 1974. "Examination of the Urine", p. 21-22 in Clinical Diagnosis by Laboratory Methods, 15th Edition, Davidson , I. & Henry, J.B. ed., W.B. Saunders, Phil. Pa.

2. Taylor, R.H. & Geldreich, E.E. 1979. A New Membrane Filer Procedure for Bacterial Counts in Potable Water and Swimming Pool Samples. J. Am. Water Works Assoc. 71, 402-405.

3. Tse, K-M and Lewis, C.M. 1984. Membrane Filter Staining Method: Bacterial Plate Counts in 24H. Applied and Environmental Microbiology 48, 433.

4. Farber, J.M. & Sharpe, A.N. 1984. Improved Bacterial Recovery by Membrane Filters in the Presence of Food Debris. Applied and Environmental Microbiology 48, 441.

5. Herlich, M.B.; Schell, R.F.; Francisco , M. and LeFrock, L. 1982. Rapid Detection of Simulated Bacteremia by Centrifugation and filtration. J. Clinical Microbiology 16, 99.

6. Lamberg, R.E.; Schell, R.F. and LeFrock, J.L. 1983. Detection and Quantitation of Simulated Anaerobic Bacteremia by Centrifugation and Filtration. J. Clinical Microbiology 17, 856.

7. Sage, B.H. Jr. and Neece, V.R. 1984. Rapid Visual Detection of Microorganisms in Blood Culture. J. Clinical Microbiology 20, 5.

8. Dorn, G.L.; Haynes, J.R. and Burson, G.G. 1976. Blood Culture Technique Based on Centrifugation: Development Phase. J. Clin. Microbiol. 3, 251.

9. Sullivan, N.M.; Finegold, S.M., and Sutter, V.L. 1975. Practical Aerobic Membrane Filtration Blood Culture Technique: Development of Procedure. J. Clin. Microbiol. 1, 30.

10. Henry, N.K., McLimans, C.A., Wright, A.J., Thompson, R.L., Wilson, W.R. and Washington, J.A. II. 1983. Microbiological and Clinical Evaluation of the Isolator Lysis-Centrifugation Blood Culture Tube. J. Clin. Microbiology 17, 864.

11. Barkley, W. Emmett, "Containment and Disinfection: in Manual of Methods for General Bacteriology, P. Gerhardt, R. G. E. Murray, R.M. Costilow, E.W. Nester, W. A. Wood, N. R. Kreig, and G. B. Philips, Editors. American Society for Microbiology, Washington, D.C. pp. 492-494, 1981.

Summary of the Invention

Culture of microorganisms is routinely carried out on semi-solid agar medium containing a wide variety of growth supporting components. Indeed, the commercial availability of a wide variety of prepared media in a standard petri dish type format enhances the ability of the microbiologist to recover and identify microorganisms. Many of the procedures routinely performed in the identification and susceptibility testing of microorganisms are dependent upon the sample containing only a single species of microorganism. The growth of isolated colonies on agar media is the simplest and most reliable means of assuring that a pure stain is being manipulated. Media with either selective or growth enhancing properties become an important part of the recovery and identification practice. Standard methods and accessories (anaerobic culture devices, plate counters, etc.) are most often designed to deal with standard plated media.

The present invention is designed to allow the use of standard plated prepared media for the growth of organisms concentrated from dilute specimens. In one embodiment of the invention, the plated media becomes a part of the device in use.

A sample of 0.10 ml is the maximum amount typically applied to plated media in accordance with the prior art. Application of greater volumes preclude the standard procedure of incubation with the covered petri type plated media in an inverted position. This inverted position combined with an inoculum of limited size are critical to obtaining individual colonies for further processing. The present invention allows samples of up to 20 cc to be applied.

In accordance with the present invention, a microorganism impermeable fluid barrier which is permeable to fluids is used in combination with liquid absorption material to separate any microorganisms in a sample from most of the liquid and to retain the microorganisms for further growth. While the concentrating effect is particularly advantageous in the processing of clinical microbiological samples, particles other than microorganisms can also be concentrated by the device.

One of the features of the invention is the use of absorbent material with a high liquid absorption capacity as the driving force for the fluid removal. Practical concentration times can not be obtained without the absorbent material. The incorporation of an absorbent material into the concentrating device also has the beneficial effect of providing a clean, convenient system with little possibility of spillage of potentially infectious or hazardous liquid. This benefit is of real value when dealing with samples that may contain a biohazard such as virus particles that are small enough to pass the microorganism impermeable barrier. Similarly, bacterial toxins may be present in the fluid which is absorbed. Thus, the device is uniquely suited for use in concentrating microorganisms even if vacuum is used to assist or accelerate the flow of liquid.

Description of the Drawings

Figure 1 is an exploded view of the concentrating device of the invention;
Figure 2 is an enlarged cross section view of the concentrating device of the invention as it would be used in combination with a prepared plate petri dish for concentration of microorganisms; and
Figure 3 is a still further enlarged view of the portion of Figure 2 founded by dotted lines.

Detailed Description of the Invention

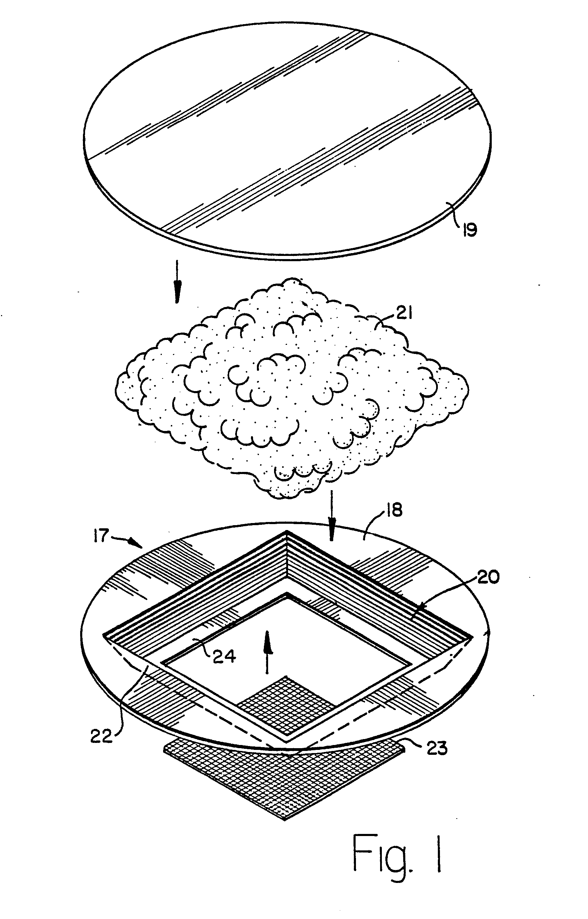

As shown in Figure 1, the concentrating device of the present invention includes a preformed body 17 which includes a peripheral flange 18 surrounding a chamber 20. The chamber 20 has depending side walls 22 and a bottom wall 24. The bottom wall 24 has an opening therein over which a membrane 23 is attached. An absorbent material 21 is placed in the chamber 20. The absorbent material 21 is in contact with the membrane 23 to cause passage of fluid through membrane 23 when membrane 23 comes in contact with a fluid. The movement of fluid is by capillary attraction from the absorbent material. A backing cover 19 may be placed over the peripheral flange 18 to contain the absorbent material in place in the chamber 20. The cover 19 may be a lid used in association with a petri dish. Alternatively, the concentrating device body 17 may be formed with a solid or pervious cover including the peripheral flange 18 over the chamber 20. The absorbent material can then be inserted through the opening in the bottom wall prior to attachment of the membrane 23.

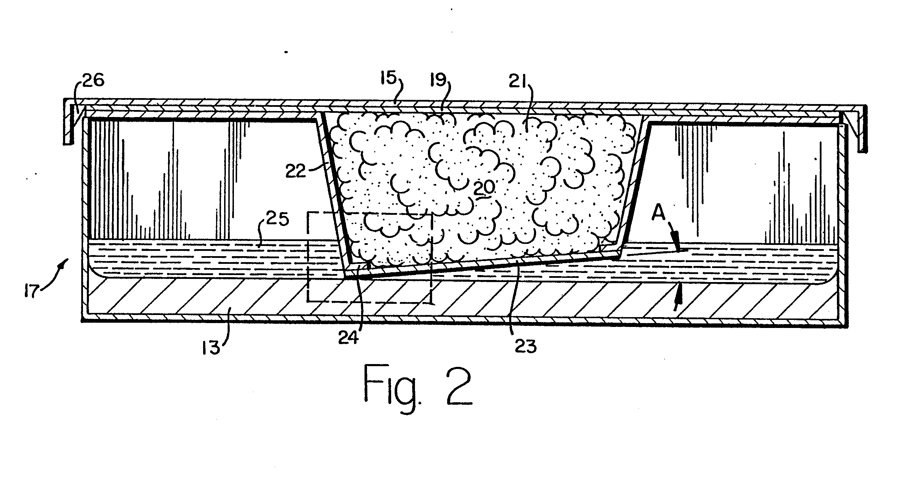

In one embodiment of the invention, as shown in figure 2, the peripheral flange 18 is configured to fit into the lid 15 of a petri dish 11. The peripheral flange 18 fits between the spacing lugs 26 which are molded into the lid 15 to provide for spacing of the lid 15 from the side wall of the petri dish 11 to permit air to pass between the space between the depending side wall of the lid 15 and the side wall of the petri dish 11. The use of spacing lugs is conventional in the manufacture of petri dishes. In the embodiment shown in Figure 2, a separate cover 19 is applied to the peripheral flange 18 prior to placing the concentrating device 17 in the petri dish lid 15.

The cross sectional shape of the chamber 20 may be any suitable configuration, such as square (as shown in Figure 1), circular (causing chamber 20 to be cylindrical in shape), rectangular or irregular.

As shown in Figure 2 the depending side walls 22 of chamber 20 are sufficiently long to locate the permeable membrane 23 in contact with a liquid sample 25 which is placed on the surface of the solid nutrient medium 13. In a preferred embodiment, a first portion of the side wall 22 is made longer than an opposing second portion of the side wall 22 whereby when the first side wall portion is located in proximity to the surface of the solid nutrient medium the membrane extends from the first side wall portion to the second side wall portion at an angle A with respect to the surface of the solid nutrient medium. This permits the first sidewall portion to be made sufficiently long to extend into firm contact with the upper surface of the solid nutrient medium 13 while providing a passage for the liquid sample to come into contact with the membrane 23. The angle A is not critical and can be any angle from 0° (when the first portion of the side wall is not made longer than the opposing second portion) to about 10°. The term "portion of the side wall" is used to accommodate the use of a cylindrical chamber 20 where only a small section of the side wall 22 would be in proximity to the solid nutrient medium 13 when angle A is made sufficiently large, such as from about 2° to about 10°.

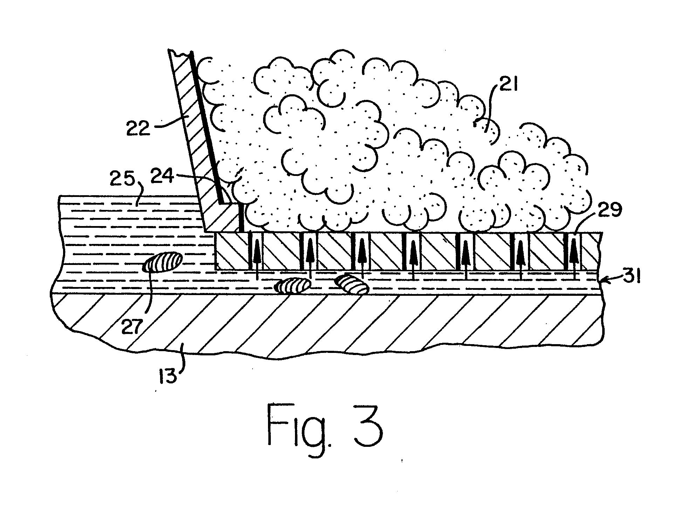

In use, an aliquot of 10 ml of a aqueous fluid to be tested for the presence of microorganisms is applied to the solid nutrient medium 13. The particle concentrating device is placed over the solid nutrient medium 13 as with a conventional lid. The membrane 23 is located to rest near or lightly on the agar surface. It is sometimes advantageous to have the membrane 23 located on at least one open side of the absorbent material 21 as well as horizontally located and completely in contact with the solid nutrient medium 13. As depicted in Figure 3, water and dissolved soluble components in the sample pass through pores 29 of the membrane 23. Bacteria 27 and other cellular or particulate material will be unable to pass through the membrane and will be deposited upon the surface of the solid medium 13. Suitable membrane materials are polycarbonate, polyester, cellulose acetate, nylon and nitrocellulose. In general, the pores have an effective diameter of from about 0.1 to about 10 microns.

After the particulate material has been concentrated on the solid surface, the concentrating device is usually removed from proximity with the solid surface and disposed of in a suitable manner.

The surface area of the membrane and the absorbency of the material used provide a relatively rapid removal of excess fluid volume. The device will remove, for example, sufficient liquid from a 10 ml sample to allow standard handling and manipulations of the inoculated petri dish within 1 to 30 minutes.

The rapid removal of excess sample volume is also desirable to minimize changes of composition of the underlying solid nutrient medium 13 by diffusion mediated exchange with the sample liquid. The growth properties should remain those of the solid nutrient medium when used with normal inoculation volumes. As long as the rate of removal of the liquid is not excessively rapid, it is believed that only a fraction of any organisms (or other particulate materials) will impinge upon the membrane and be at risk to absorption or passage loss. As described in the examples, results have shown good recovery of microorganisms. The loose absorption or binding of a fraction of the available microorganisms to the membrane layer has been exploited by utilizing the device to inoculate a second (and, at times, different) solid nutrient medium surface. Merely pressing or blotting the device to the surface of the second plate transfers most organisms from the membrane surface to the agar growth media.

The removal of the great majority of the water soluble components, such as an antibiotic present in the sample, represents a partial purification of the organisms present. Further purification may be carried out by addition of an aliquot of sterile liquid to the once concentrated plate and reconcentration in the same manner.

Absorbent material can be chosen from a variety of materials with a high affinity and capacity for water: e.g., Sephadex, cellulosic and modified cellulosic materials, or polymeric acrylamide derivatives. Diatomaceous earth has been shown to be a particularly preferred material for this application. The inorganic nature of diatomaceous earth assures low toxicity and allows heat and radiation sterilization with little concern for deterioration.

## Examples

Table 1 lists the time required to draw 10 ml. through a 0.8 micron membrane when a variety of different absorbent materials were utilized. These materials include synthetic polymer (Bio-Gel™ P-6; a beaded polyacrylamide/methylene-bis-acrylamide copolymer), a natural polymer (Sephadex™ G-25, a crosslinked dextran), and a commercially available absorbent cotton type fibre (cellulose fiber from Always™ brand mini-pads).

Examples 2, 3 and 4 summarize the use of the concentrating device for the concentration of artificially prepared microorganism suspension in a typically commercially available liquid growth medium (BBL Trypticase® Soy Broth Catalog #21480). The final samples had an expected average bacterial load of less than 10 CFU (colony forming units) per ml.

Each plate prepared with a sample of 10 ml using the prototype devices had multiple colonies. A second plate was inoculated with the device after the sample had been concentrated by a) removal from the first plate and b) gently blotting the membrane portion of the device on the surface of the second plate. Tables 2-5 summarize this data. Table 5 is a summary of data that lists, from all experiments, the number of colonies recovered on each plate utilized. This clearly shows that use of the concentrating device of the invention provides useful numbers of isolated colonies in all cases while the standard protocol provided more than two colonies in only 1 of 50 trials.

Standard plating procedures with 0.1 ml samples yielded no detectable colonies in the majority of cases. This is not surprising, since in some cases, the anticipated "average" number of CFU's present in the sample would be fractional. Fractional parts of an organism would not be viable --ie. no growth is expected in many of these sample.

The device of the invention is not limited to blood culture samples. Water samples are routinely analyzed by water treatment and supply companies to determine the number of organisms present. This is another appropriate application. Similarly, the combination of a barrier impervious to microorganisms with an absorbent material could be applied to a tube type device to concentrate a dilute sample prior to application to a growth media or to merely provide a non-vacuum source for filtration prior to microscopic examination. The detailed description of the direct concentration of dilute samples onto the surface of a standard plated growth media is a unique invention particularly when combined with the absence of vacuum as a driving force for the fluid migration.

Fine latex particles of one to three micron diameter are often reacted to form derivative products of use in immunoassay procedures. The device of the invention would be useful in concentrating and purifying such reacted latex. In such an application, a plastic container would be substituted for the solid nutrient media and the product would be a liquid solution of concentrated latex particles. This is only one of many non-biological applications.

The concentrating devices of the invention can also operate with vacuum as the driving force for fluid migration across the membrane. Addition of a fibrous layer in front of a small surface area device would produce a pencil like combined concentrator/inoculator (swab like) device.

## Example One

## Concentration Using Various Absorbent Materials

Square concentrator prototypes (surface area = 1.60 in² using a 0.8 micron Nuclepore® (PLC-AB) membrane were used to test the absorptive capacity of various materials in drawing up 10 ml of Trypticase® Soy Broth (BBL #21480) after a 20 minute time period.

## Table 1

### Concentration With Various Absorbents*

| Material Tested (Amt Grams) | Weight of Empty Plate | Total Weight Plate+10ml TSB | Weight @20 min | %Sample Removed |
|---|---|---|---|---|
| Sephadex<sup>R</sup> G-25 Fine (3.0) | 32.66 | 42.66 | 32.72 | 99.4 |
| Bio-Gel<sup>R</sup> P-6 (2.75) | 32.82 | 42.82 | 33.28 | 95.4 |
| Manville Super-Cel<sup>TM</sup> Diatomaceous Earth (6.5) | 32.42 | 42.42 | 32.70 | 97.2 |
| Cellulose Fiber (3.0) | 33.29 | 43.29 | 33.82 | 94.7 |
| Empty Device | 32.49 | 42.49 | 42.29 | 2.0 |

*Note:

% Sample Removed = [10 - (Weight at 20 min - Weight Empty)] x 10

Cellulose Fiber - found in Procter & Gamble Always™ Mini Pads

Empty device - tested to identify the natural flow through a Nuclepore™ membrane

All of the materials tested greatly accelerated the movement of the media through the membrane. At least 94% of the applied material had been removed by the device within twenty minutes.

Example 2

Concentrating Device Biological Study

Purpose:

Verify the ability of the concentrating device to isolate bacteria at very low concentrations.

Procedure:

1. Two vials of Staphylococcus aureus (ATTC #25923) are thawed and suspended in separate 8 ml tubes of Trypticase™ Soy Broth and incubated at 37° for 24 hours.

2. Put 3.0 ml. of the bacteria culture into a clean disposable cuvette (path = 1.0 cm.).

3. Measure the transmission of the broth culture on a spectrophotometer set at wavelength of 660 and slit of 0.6 mm. The machine should be zeroed using fresh Trypticase™ Soy Broth.

4. Adjust the broth culture to read at 52% transmission by slowly adding clean broth at 200 ul/time and measuring the transmission after each adjustment.

5. Add 1.0 ml of adjusted broth culture to 9.0 ml physiologically buffered saline (PBS) (1/10) dilution). Label as DILUTION I.

6. Add 90 ul dilution I to 8910 ul PBS (1/100 dilution). Label as DILUTION II.

7. Add 90 ul Dilution II to 8910 ul PBS (1/100 dilution). Label as DILUTION III.

8. Add 1.0 ml of Dilution III to 9.0 ml PBS (1/10 dilution). Label as DILUTION IV.

9. Inoculate ten g Trypticase Soy Agar w/5 % sheep's blood plates with 0.1 ml Dilution IV. This set of plates will be used to calculate the concentration of bacteria added in subsequent steps.

10. Add 600 ul of Dilution IV to 59.4 ml (weighed out) PBS (1/100 dilution). Label as DILUTION V.

11. Inoculate five g Trypticase Soy Agar w/5% sheep blood plates with 10.0 ml of Dilution V.

12. Place a sterile prototype concentrating device onto each plate and allow it to absorb for 15 minutes or until all visible liquid around the device is absorbed.

13. Remove the prototype concentrating device and spread any remaining liquid with a sterile glass spreader.

14. Carefully blot the used prototype concentrating device onto an appropriately labeled fresh plate. Keep track of which plates correspond to which prototype concentrating device.

15. Inoculate twenty g Trypitcase Soy Agar w/5% sheep's blood plates with 0.1 ml of dilution V. These plates will be directly compared to the prototype concentrating device plates.

16. Incubate all plates at 37°C for 24 hours and count all colonies.

Results:

The results obtained by carrying out this procedure with prototype concentrating devices produced with 0.8 micron Nuclepore® membrane and 3.0 gm. of fiber from Always® product are summarized in Table 2 and included with other experiments in Table 5.

Example 3

Concentrating Device Biological Study

Purpose:

Verify the ability of the concentrating device to isolate bacteria at very small concentrations.

Procedure:

The procedure utilized was essentially that described in Example 2 (above).

Results:

The results of this example are shown in Table 3 and Table 5.

Example 4

Concentrating Device Biological Study

Purpose:

Verify the ability of the concentrating device to isolate bacteria at very small concentrations.

Procedure:

The procedure utilized was essentially that described in Example 2 (above) except that Escherichia coli (ATTC Culture $25922) was utilized. A Transmittance of 30% was found to give appropriate concentrations of organisms.

ml samples of Dilution IV plated and counted as described in Example 2.

## TABLE 3

### EXAMPLE 3 MICROBIOLOGICAL CONCENTRATION DATA

| | Sample | Concentrated CPU*** | | CPU Found | | |
|---|---|---|---|---|---|---|
| | | Y (Yes) or | | | | |
| # | (ml) | N (No) | Expected | Plate 1 | | Plate 2 |
| Total | | | | | | |
| 1 | 0.1 | N | 0.395 | 1 | | 1 |
| 2 | 0.1 | N | 0.395 | 0 | | 0 |
| 3 | 0.1 | N | 0.395 | 0 | | 0 |
| 4 | 0.1 | N | 0.395 | 0 | | 0 |
| 5 | 0.1 | N | 0.395 | 0 | | 0 |
| 6 | 0.1 | N | 0.395 | 1 | | 1 |
| 7 | 0.1 | N | 0.395 | 1 | | 1 |
| 8 | 0.1 | N | 0.395 | 1 | | 1 |
| 9 | 0.1 | N | 0.395 | 0 | | 0 |
| 10 | 0.1 | N | 0.395 | 1 | | 1 |
| 11 | 0.1 | N | 0.395 | 1 | | 1 |
| 12 | 0.1 | N | 0.395 | 0 | | 0 |
| 13 | 0.1 | N | 0.395 | 0 | | 0 |
| 14 | 0.1 | N | 0.395 | 0 | | 0 |
| 15 | 0.1 | N | 0.395 | 2 | | 2 |
| 16 | 0.1 | N | 0.395 | 1 | | 1 |
| 17 | 0.1 | N | 0.395 | 0 | | 0 |
| 18 | 0.1 | N | 0.395 | 2 | | 2 |
| 19 | 0.1 | N | 0.395 | 0 | | 0 |
| 20 | 0.1 | N | 0.395 | 0 | | 0 |
| | | Average | 0.395 | 0.60 | | 0.60 |
| 1 | 10.0 | Y | 39.5 | 14 | 12 | 26 |
| 2 | 10.0 | $\bar{\bar{Y}}$ | 39.5 | 14 | 11 | 33 |
| 3 | 10.0 | Y | 39.5 | 13 | 21 | 22 |
| 4 | 10.0 | Y | 39.5 | 12 | 13 | 25 |
| 5 | 10.0 | Y | 39.5 | 17 | 4 | 21 |
| | | Average | 39.5 | 14.0 | 12.2 | 26.2 |

Results:

The results of example 4 are summarized in Table 4 and Table 5.

## TABLE 2

### EXAMPLE 2 MICROBIOLOGICAL CONCENTRATION DATA

| Sample # | Concentrated (ml) | CPU** Y (Yes) or N (No) | Expected | Plate 1 | Plate 2 | CPU Pound Total |
|---|---|---|---|---|---|---|
| 1 | 0.1 | N | 0.28 | 0 | | 0 |
| 2 | 0.1 | N | 0.28 | 0 | | 0 |
| 3 | 0.1 | N | 0.28 | 0 | | 0 |
| 4 | 0.1 | N | 0.28 | 1 | | 1 |
| 5 | 0.1 | N | 0.28 | 0 | | 0 |
| 6 | 0.1 | N | 0.28 | 2 | | 2 |
| 7 | 0.1 | N | 0.28 | 0 | | 0 |
| 8 | 0.1 | N | 0.28 | 1 | | 1 |
| 9 | 0.1 | N | 0.28 | 0 | | 0 |
| 10 | 0.1 | N | 0.28 | 0 | | 0 |
| 11 | 0.1 | N | 0.28 | 0 | | 0 |
| 12 | 0.1 | N | 0.28 | 0 | | 0 |
| 13 | 0.1 | N | 0.28 | 0 | | 0 |
| 14 | 0.1 | N | 0.28 | 0 | | 0 |
| 15 | 0.1 | N | 0.28 | 0 | | 0 |
| 16 | 0.1 | N | 0.28 | 0 | | 0 |
| 17 | 0.1 | N | 0.28 | 1 | | 1 |
| 18 | 0.1 | N | 0.28 | 0 | | 0 |
| 19 | 0.1 | N | 0.28 | 2 | | 2 |
| 20 | 0.1 | N | 0.28 | 0 | | 0 |
| | | Average | 0.28 | 0.35 | | 0.35 |
| 1 | 10.0 | Y | 28 | 15 | 9 | 24 |
| 2 | 10.0 | Y | 28 | 21 | 12 | 33 |
| 3 | 10.0 | Y | 28 | 9 | 13 | 22 |
| 4 | 10.0 | Y | 28 | 16 | 21 | 37 |
| 5 | 10.0 | Y | 28 | 9 | 10 | 19 |
| | | Average | 28 | 14.0 | 13.0 | 27.0 |

** This number was calculated from 20 replicate 0.10

9

*** This number was calculated from 20 replicate 0.10 ml samples of Dilution IV plated and counted as described in Example 2.

## TABLE 4

### EXAMPLE 4 MICROBIOLOGICAL CONCENTRATION DATA

| # | (ml) | Y (Yes) or N (No) | Concentrated CFU**** Expected | CFU Found Plate 1 | Plate 2 | Total |
|---|------|------|----------|---------|---------|-------|
| 1 | 0.1 | N | 0.989 | 0 | | 0 |
| 2 | 0.1 | N | 0.989 | 2 | | 2 |
| 3 | 0.1 | N | 0.989 | 0 | | 0 |
| 4 | 0.1 | N | 0.989 | 4 | | 4 |
| 5 | 0.1 | N | 0.989 | 0 | | 0 |
| 6 | 0.1 | N | 0.989 | 2 | | 2 |
| 7 | 0.1 | N | 0.989 | 2 | | 2 |
| 8 | 0.1 | N | 0.989 | 2 | | 2 |
| 9 | 0.1 | N | 0.989 | 1 | | 1 |
| 10 | 0.1 | N | 0.989 | 1 | | 1 |
| | | Average | 0.989 | 1.40 | | 1.40 |
| 1 | 10.0 | Y | 98.9 | 26 | 19 | 45 |
| 2 | 10.0 | Y | 98.9 | 38 | 12 | 50 |
| 3 | 10.0 | Y | 98.9 | 42 | 19 | 61 |
| | | Average | 98.9 | 35.3 | 16.6 | 52.0 |

**** This number was calculated from 20 replicate 0.10 ml samples of Dilution IV plated and counted as described in Example 2.

## TABLE 5 COMBINED DATA

| #CFU Range | Device Plate 1(n=13) | Device Plate 2(n=13) | Both Plates | Control (0.1 ml) |
|---|---|---|---|---|
| 0 | 0 | 0 | 0 | 29 |
| 1-2 | 0 | 0 | 0 | 20 |
| 3-8 | 0 | 1 | 0 | 1 |
| 9-14 | 6 | 8 | 0 | 0 |
| 15-20 | 3 | 2 | 6 | 0 |
| 21-30 | 2 | 2 | 6 | 0 |
| 31-40 | 2 | 0 | 3 | 0 |
| 41+ | 0 | 0 | 3 | 0 |

Claims

Claim 1. A device for concentration of particles present in a fluid sample located on the surface of a solid material comprising a body having a peripheral flange surrounding a depending chamber, said chamber having side walls and a bottom wall having an opening therein, an absorbent material disposed within said chamber, a membrane disposed over said opening in said bottom wall, said membrane being previous to fluids but is impervious to the particles and means for locating said membrane in proximity to the surface of said solid material.

Claim 2. A device in accordance with Claim 1 wherein said particles are microorganisms, and said solid material is a solid nutrient medium disposed in a petri dish.

Claim 3. A device in accordance with Claim 2 wherein said peripheral flange is configured to fit into a mating lid for said petri dish.

Claim 4. A device in accordance with Claim 2 wherein said peripheral flange is configured so that said peripheral flange extends over the side wall of said petri dish.

Claim 5. A device in accordance with Claim 1 wherein a first portion of said side wall is longer in combination with an opposing second portion of said side wall causes said membrane extending from said first side wall portion to said second side wall portion to form an angle A with respect to said surface of said solid nutrient medium when said first side wall portion is located in proximity to said surface.

Claim 6. A device in accordance with Claim 5 wherein said angle A is from about 0° to about 10°.

7. A device in accordance with Claim 1 wherein said membrane has a pore size of from about 0.1 to about 1.0 microns.

Claim 8. A device in accordance with Claim 1 wherein said absorbent material is selected from the group consisting of diatomaceous earth, cellulose fibers, polymeric gels and dextran.

Claim 9. A method for concentration of particles present in a fluid sample located on the surface of a solid material comprising providing a chamber having a membrane affixed to an opening therein and an absorbent material filling said chamber and locating said membrane in proximity to said surface of said solid material whereby said fluid is caused to pass through said membrane and be absorbed by said absorbent material.

Claim 10. A method in accordance with Claim 9 wherein said particles are microorganisms and said solid material is a solid nutrient medium disposed in a petri dish.

Claim 11. A method in accordance with Claim 10 wherein said chamber is provided with a peripheral flange configuration such that said peripheral flange fits into a mating lid for said petri dish.

Claim 12. A method in accordance with Claim 10 wherein said chamber is provided with a peripheral flange configuration such that said peripheral flange extends over the side wall of said petri dish.

Claim 13. A method in accordance with Claim 9 wherein said membrane has a said pore size from about 0.1 to about 1.0 microns.

Claim 14. A method in accordance with with Claim 9 wherein said absorbent material is selected from the group consisting of diatomaceous earth, cellulose fibers, polymeric gels and dextran.

Fig. 1

Fig. 2

0 261 676

Fig. 3

0 261 676